# EUROPEAN PATENT SPECIFICATION

(11) **EP 3 132 072 B1**
(45) Date of publication and mention of the grant of the patent: **04.12.2019**
(21) Application number: 15780044.2
(22) Date of filing: 17.04.2015
(51) Int. Cl.: C12N 15/10, G01N 15/10, B01J 19/00, C12N 15/64, C12N 15/66, C40B 50/10, C12Q 1/6869, C12Q 1/68

(54) **METHODS FOR DROPLET TAGGING**
VERFAHREN ZUR TRÖPFCHENMARKIERUNG
PROCÉDÉS D'ÉTIQUETAGE DES GOUTTELETTES

(30) Priority: 17.04.2014 US 201461981123 P
(43) Date of publication of application: 22.02.2017
(73) Proprietor: President and Fellows of Harvard College, Cambridge, MA 02138 (US); The Broad Institute, Inc., Cambridge, MA 02142 (US); The General Hospital Corporation d/b/a Massachusetts General Hospital, Boston, MA 02199-8001 (US)
(72) Inventor: BERNSTEIN, Bradley, E., Cambridge, MA 02138 (US); NICOL, Robert, Cambridge, MA 02142 (US); WEITZ, David, A., Bolton, MA 01740 (US)
(74) Representative: Hoffmann Eitle
(86) International application number: PCT/US2015/026338
(87) International publication number: WO 2015/161177

(56) References cited:
- WO-A1-2009/085215
- WO-A1-2013/134261
- WO-A1-2013/134261
- WO-A1-2014/047561
- WO-A2-2010/025310
- US-A1- 2011 033 854
- US-A1- 2012 220 494

## Description

### RELATED APPLICATIONS

This application claims the benefit of U.S. Provisional Patent Application Serial No. 61/981,123, filed April 17, 2014, entitled "Systems and Methods for Droplet Tagging," by Bernstein, *et al..*

### GOVERNMENT FUNDING

This invention was made with government support under Grant No. P01GM096971 awarded by the National Institutes of Health, and under Grant Nos. DMR-1006546 and DMR-0820484 awarded by the National Science Foundation. The government has certain rights in the invention.

### FIELD

The present invention generally relates to microfluidic devices, including systems and methods for tagging droplets within such devices.

### BACKGROUND

A variety of techniques exist for producing fluidic droplets within a microfluidic system, such as those disclosed in Int. Pat. Pub. Nos. WO 2004/091763, WO 2004/002627, WO 2006/096571, WO 2005/021151, WO 2010/033200, and WO 2011/056546. In some cases, relatively large numbers of droplets may be produced, and often at relatively high speeds, e.g., the droplets may be produced at rates of least about 10 droplets per second. The droplets may also contain a variety of species therein. However, it can be difficult to accurately track such droplets, especially when large numbers of droplets are produced and/or the droplets are produced at very high rates. In addition, such tracking may be complicated if the droplets are exposed to a variety of different conditions, contain different species, etc., such that the droplets are not all identical.

### SUMMARY

The present invention is defined by the claims. The present description generally relates to microfluidic devices, including systems and methods for tagging droplets within such devices. The subject matter of the present description involves, in some cases, interrelated products, alternative solutions to a particular problem, and/or a plurality of different uses of one or more systems and/or articles.

WO 2009/085215 A1 concerns systems and methods for nucleic acid sequencing.

WO 2010/025310 A2 concerns methods and devices for high fidelity polynucleotide synthesis.

US 2011/033854 A1 concerns methods and compositions for long fragment read sequencing.

WO 2014/047561 A1 concerns compositions and methods for labeling of agents.

WO 2013/134261 A1 concerns systems and methods for epigenetic sequencing.

In one aspect, the present description is generally directed to a method, According to one set of embodiments, the method comprises exposing a droplet to a first condition and adding a first nucleic acid to the droplet, wherein the first nucleic acid encodes the first condition; exposing the droplet to a second condition and adding a second nucleic acid to the droplet, wherein the second nucleic acid encodes the second condition; and ligating the first nucleic acid and the second nucleic acid together.

The method, in accordance with another set of embodiments, includes can act of exposing a plurality of droplets to a plurality of conditions such that substantially each droplet is sequentially exposed to at least two different conditions, wherein when a droplet is exposed to a condition, a nucleic acid encoding the condition is added to the droplet.

In yet another set of embodiments, the method includes acts of exposing a cell contained within a droplet to a first species, and adding a first nucleic acid to the droplet, exposing the cell to a second species, and adding a second nucleic acid to the droplet, ligating the first nucleic acid and the second nucleic acid together, determining a property of the cell, and sorting the droplet based on the property of the cell.

Still another set of embodiments is generally directed to a method comprising an act of encapsulating a plurality of cell types and a plurality of nucleic acids in a plurality of droplets such that substantially each droplet comprises one or more cells of only one cell type and an encoding nucleic acid encoding the only one cell type.

The method, in one set of embodiments, includes acts of providing a plurality of cell types and a plurality of nucleic acids in a plurality of droplets such that substantially each droplet comprises one or more cells of only one cell type and an encoding nucleic acid encoding the only one cell type, lysing the cells within the droplets to release cellular nucleic acid from the cells, ligating the encoding nucleic acid and the cellular nucleic acid together, and sequencing the ligated nucleic acids.

In another set of embodiments, the method includes acts of providing a plurality of cell types and a plurality of nucleic acids in a plurality of droplets such that substantially each droplet comprises one or more cells of only one cell type and an encoding nucleic acid encoding the only one cell type; exposing the plurality of droplets to a plurality of species such that substantially each droplet is exposed to a single species of the plurality of species, and adding a first nucleic acid encoding the single species thereto; lysing the cells within the droplets to release cellular nucleic acid from the cells; ligating the encoding nucleic acid, the first nucleic acid, and the cellular nucleic acid together; and sequencing the ligated nucleic acids.

In another aspect, the present description is generally directed to a plurality of droplets. In some cases, at least some of the droplets contain nucleic acids encoding a plurality of conditions that the at least some droplets were exposed to.

The present description in accordance with another aspect, is generally directed to a library of droplets containing a plurality of cell types and a plurality of nucleic acids. In some embodiments, substantially each of the cell types is encoded by a unique nucleic acid sequence.

In another aspect, the present description encompasses methods of making one or more of the embodiments described herein. In still another aspect, the present description encompasses methods of using one or more of the embodiments described herein.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
Figs. 1A-1C schematically illustrate various methods for tagging droplets, in accordance with various embodiments of the invention; and
Figs. 2A-2B schematically illustrates joining together various nucleic acid tags, in certain embodiments of the invention.

### DETAILED DESCRIPTION

The present description generally relates to microfluidic devices, including systems and methods for tagging droplets within such devices. In some aspects, microfluidic droplets are manipulated by exposing the droplets (or other entities) to a variety of different conditions. By incorporating a plurality of "tags," e.g., nucleic acid tags, into the droplets and joining the tags together, the conditions that a droplet was exposed to may be encoded. Thus, even if droplets exposed to different conditions are mixed together, the conditions that each droplet encountered may still be determined, for example, by sequencing the nucleic acids.

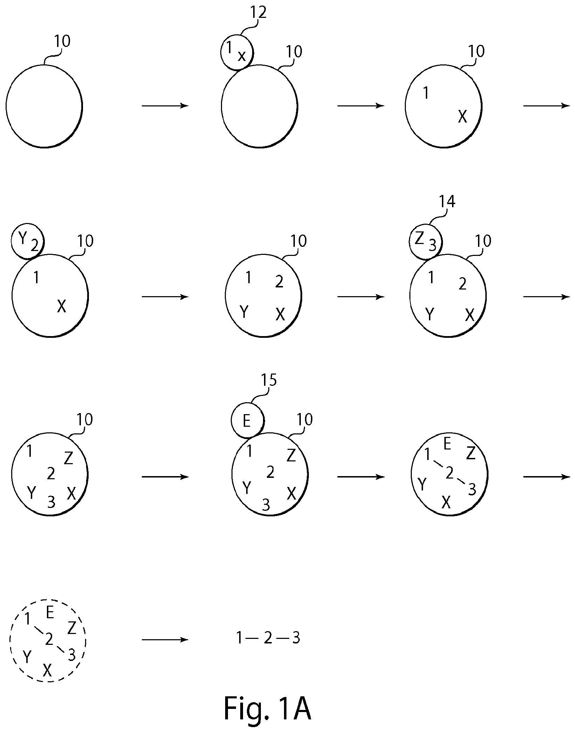

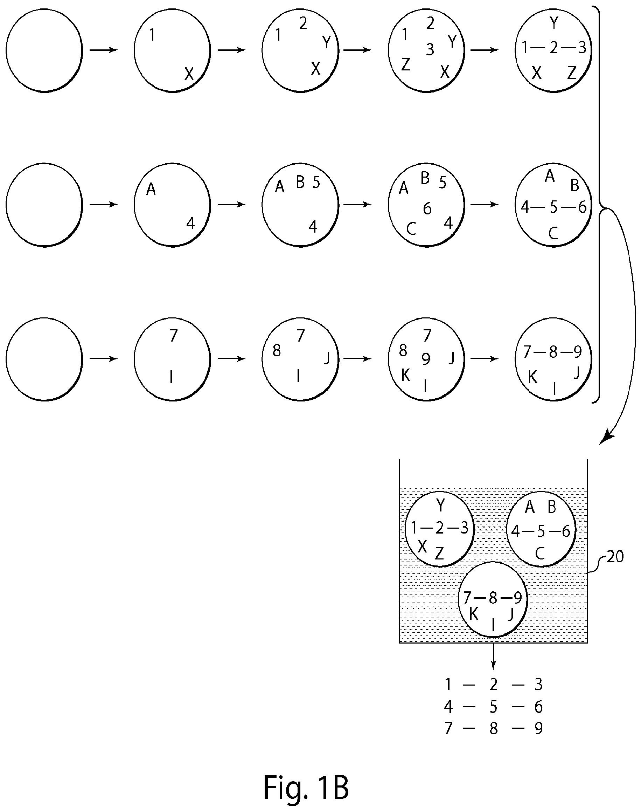

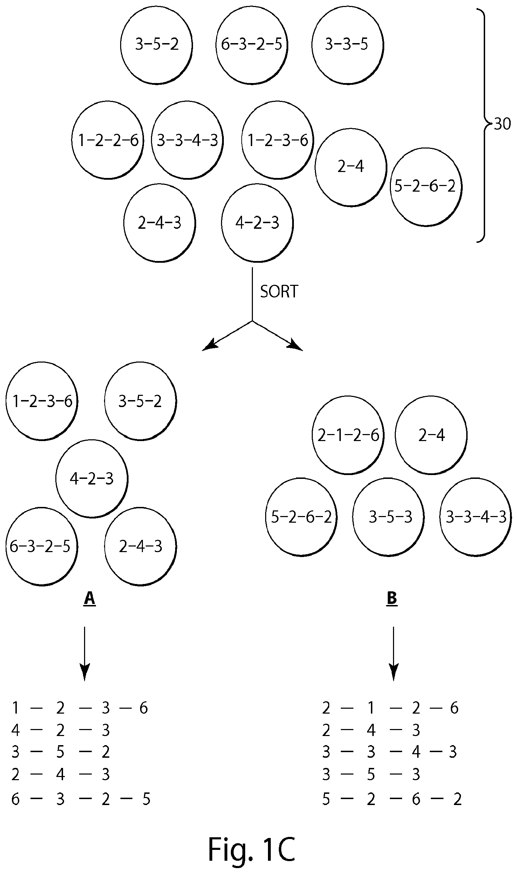

Initially, certain non-limiting examples are discussed with reference to Fig. 1. However, in other embodiments, other configurations may be used as well. Turning first to Fig. 1A, a droplet is shown that is exposed to a variety of conditions (for example, a species such as a drug), and each time the droplet is exposed to a condition, a nucleic acid encoding the condition is added to the droplet. It should be noted that the condition need not be the addition of a chemical species, but could also be a physical condition, such as exposure to a particular temperature. It should further be noted that although droplets are discussed with reference to Fig. 1, this is for ease of presentation only; in other embodiments, other discrete entities may be used instead of these droplets, for example, microwells in a microwell plate.

As is shown in this example, droplet 10 first encounters, and is fused with, droplet 12 containing species X and nucleic acid 1. Subsequently, droplet 10 encounters and is fused with droplet 13 (containing species Y and nucleic acid 2) and droplet 14 (containing species Z and nucleic acid 3). An enzyme E may then be introduced in some fashion into droplet 10, for example, as part of another droplet fusion event (as is shown here with droplet 15), or the enzyme may initially be found in any one of droplets 10, 12, 13, or 14. The enzyme may be used to ligate nucleic acids 1, 2, and 3 together, as is shown with ligated nucleic acids 1-2-3. The droplet can then be burst to access the ligated nucleic acid, and the nucleic acid can be sequenced or otherwise determined to determine the history of droplet 10.

In some embodiments, two or more droplets may be treated in such a fashion, as is shown in Fig. IB. For instance, two or more droplets may be exposed to a variety of different conditions, where each time a droplet is exposed to a condition, a nucleic acid encoding the condition is added to the droplet. However, even if such droplets with different histories are later combined (e.g., in a mixture), as is shown in container 20, the conditions each of the droplets was exposed to is still determinable through the different nucleic acids contained in each droplet. This is true even if the droplets themselves are burst and/or the contents are mixed together. For example, as is shown in Fig. 1B, by determining nucleic acids 1-2-3, 4-5-6, and 7-8-9, one knows that at least one droplet in container 20 was exposed to conditions A, B, and C, at least one droplet was exposed to conditions I, J, and K, and at least one droplet was exposed to conditions X, Y, and Z. In addition, one may also be able to determine that no droplets in container 20 was exposed to other sets of conditions. For example, one may be able to determine that no droplet was exposed to conditions X, Y, and K, as there is no sequence 1-2-9 present in container 20.

As is shown in Fig. 1C, in certain embodiments of the invention, testing and/or sorting of the droplets may also occur. As shown in this figure, a plurality of droplets exposed to different conditions (as encoded by the nucleic acids contained therein, represented by various numbers) may be sorted based on some criteria. For example, if the droplet further contain cells, then a property of the cell (e.g., whether the cell is alive or dead) may be used to sort the droplets into 2 (or more) populations. Each population can then be separately analyzed, e.g., by determining the nucleic acids contained therein, to determine which conditions may have led to the different populations. For instance, in Fig. 1C, a population of droplets 30 was sorted into a first group (A) and a second group (B). The nucleic acids in each group can then be sequenced. Thus, for example, it may be determined that the members of Group A each have conditions 2 and 3 in common, while the members of Group B do not have conditions 2 and 3 in common (although conditions 2 or 3 may be separately present in some of the Group B droplets). Thus, these sorting experiments would demonstrate that the combination of conditions 2 and 3 is necessary for some effect to occur (Group A), and if both are not present, the effect does not occur (Group B).

Also noteworthy is that in Fig. 1C, the number of conditions each droplet is exposed to is not necessarily the same; instead, the conditions may vary, i.e., intentionally, or due to experimental errors or uncertainty. For example, droplet 2-4 was exposed to conditions 2 and 4, while droplet 1-2-3-6 was exposed to conditions 1, 2, 3, and 6. Furthermore, it should also be noted that, due to the ligation of the nucleic acids prior to removing the nucleic acids from the droplets, the conditions each droplet was exposed to may be separately maintained and analyzed, even if the droplets and/or the nucleic acids are subsequently mixed together. Thus, with reference to Fig. 1C, if the nucleic acids were not ligated together, then each of Groups A and B would appear to be identical, since each group contains the same numbers (representing conditions) in exactly the same proportions in this illustrative example.

Thus, one surprising feature of certain embodiments of the invention is that the ligated nucleic acids allow more complex conditions to be readily determined, for example a single condition or multiple conditions. This can be accomplished without loss of information even if the droplets are burst or otherwise combined together, e.g., for ease of processing or analysis. In contrast, in techniques in which various tags are separately introduced into droplets, without ligation or other methods of combining the tags together, the conditions cannot be so readily analyzed; instead, care must be taken in keeping each of the droplets separate, as any tags that are accidently combined (for example by bursting or fusing different droplets together) would result in loss of information.

The above discussion is not intended to be limiting; other embodiments of the invention are also possible for tagging droplets, as will now be discussed. For instance, various aspects of the present description are generally directed to systems and methods for tagging or identifying droplets within microfluidic devices, e.g., using nucleic acids and other "tags," that can be bound together. By binding the tags together, information about the droplet containing the tag may be retained, e.g., even after the tag is separated from the droplet and/or combined with other, different tags. Thus, for example, a plurality of tags from different droplets (e.g., exposed to different conditions) may be combined and analyzed together.

Certain aspects of the present description involve the use of a plurality of droplets or other discrete entities, e.g., where the contents of the entities are not readily mixed with the contents of other entities. For example, the discrete entities may be droplets contained within a carrying fluid, microwells of a microwell plate, individual spots on a slide or other surface, or the like. As discussed herein, each of the entities may a specific location that can contain one or more tags or other species, without accidental mixing with other entities. The entities may be relatively small in some cases, for example, each entity may have a volume of less than about 1 ml, less than about 300 microliters, less than about 100 microliters, less than about 30 microliters, less than about 10 microliters, less than about 3 microliters, less than about 1 microliter, less than about 500 nl, less than about 300 nl, less than about 100 nl, less than about 50 nl, less than about 30 nl, or less than about 10 nl.

In some embodiments, the droplet or other entity may contain various species, e.g., cells, chemicals, or the like. Other examples of species are discussed herein. For example, if the droplet or other entity contains one or more cells, the cells may be substantially identical or different. For example, a droplet or other entity may contain more than one cell or other species), where the cells (or other species) are the same or different; the cells (or other species) in different droplets or entities may also be the same or different. If cells are used, the cells may also be, in some embodiments, from a specific population of cells, such as from a certain organ or tissue (e.g., cardiac cells, immune cells, muscle cells, cancer cells, etc.), cells from a specific individual or species (e.g., human cells, mouse cells, bacteria, etc.), cells from different organisms, cells from a naturally-occurring sample (e.g., pond water, soil, etc.), or the like.

Thus, as non-limiting examples, the effects of one or more conditions on a specific type of cell (or specific types of cells) may be studied. As another example, the effects of a certain specific condition (or conditions) on a suitable population of cells may be studied. It addition, it should be noted that the present invention is not limited to only study of cells. In other embodiments, for example, the droplets or other entities may contain species in which a variety of conditions is to be applied. For example, the species may be a chemical reagent, e.g., one that is biological or nonbiological, organic or inorganic, etc. For instance, the species may be a polymer, a nucleic acid, a protein, a drug, a small molecular compound (e.g., having a molecular weight of less than about 1000 Da or less than about 2000 Da), an antibody, an enzyme, a peptide, or the like. Other examples of species are discussed in more detail below.

One or more tags may be present within a droplet (or other entity), which can be analyzed to determine the identity and/or history of the droplet. In some cases, the tags may be chosen to be relatively inert relative to other components of the droplet or other entity. The tags may be present initially in the droplet or other entity, and/or subsequently added, e.g., using processes such as those described below. For instance, tags may be added when the droplet or other entity is exposed to one or more conditions (or proximate in time to such exposure). In some cases, more than one tag may be present in a droplet or other entity.

In certain embodiments of the invention, the tags within a droplet or other entity can be joined together, for example, chemically, to produce a joined tag. Any suitable technique may be used to join tags together, e.g., prior to removal from the droplet or entity. The tags may be joined using any suitable technique. For example, the tags may be joined together using an enzyme, a catalyst, or a reactant, which may be added to the droplet or other entity using any suitable technique. For instance, a droplet containing the tags may be fused to another droplet containing the chemical agent, or a chemical reactant may be added or inserted into a droplet or other entity, for example, using pipetting or other techniques, and in some cases, using automated techniques.

By joining the tags in a droplet (or other entity) together to produce a joined tag, the identity and/or history of the droplet may be maintained by maintaining the joined tags, even if the tags are separated from the droplet or tags from different droplets are mixed together. For example, joined tags from a variety of droplets or entities can be collected together and analyzed. In some embodiments, a series of droplets or other entities may be separated into various groups depending on various properties, and the tags within each group may be manipulated together and/or used to identify such droplets or entities having such properties.

The tags may include, for example, nucleic acids, which may be joined together or ligated using suitable enzymes able to ligate the nucleic acids together, such as ligases. Non-limiting examples of ligases include DNA ligases such as DNA Ligase I, DNA Ligase II, DNA Ligase III, DNA Ligase IV, T4 DNA ligase, T7 DNA ligase, T3 DNA Ligase, *E. coli* DNA Ligase, Taq DNA Ligase, or the like. Many such ligases may be purchased commercially. In addition, in some embodiments, two or more nucleic acids may be ligated together using annealing or a primer extension method.

As discussed herein, various sequences of nucleic acids can be used to encode specific conditions that a droplet or other entity may be exposed to, and such nucleic acids can be added thereto to indicate such exposure to a condition, in accordance with certain embodiments. In some cases, the nucleic acids within a droplet or other entity may be ligated together prior to removal (for example, upon bursting of a droplet, washing of a slide, etc.). Different nucleic acids from different droplets or entities may be mixed together; however, even after such mixing, each nucleic acid can be individually sequenced to determine the specific conditions that the corresponding droplet or entity had been exposed to.

Any suitable system may be used for encoding. For example, in one set of embodiments, a nucleic acid tag may include an encoding region of nucleotides, and optionally a connecting region. The nucleotides in the encoding region may correspond to a specific condition (or set of conditions). Any suitable number of conditions may be arbitrarily encoded in such a fashion, where the number of conditions that are encodable by such an encoding region may be determined by the number of nucleotides in the encoding region. Thus, for instance, an encoding region having length n can encode up to 4" regions (based on the four types of nucleotides). For example, a first condition may be encoded with A, a second condition may be encoded with T (or U if the nucleic acid is an RNA), a third condition may be encoded with G, a fourth condition may be encoded with C, etc. As a more complex example, an encoding region containing 3 nucleotides is sufficient to encode over 50 different conditions (since 4³ = 64). One or more than one encoding region may be used. In addition, the encoding region may also include other nucleotides used for error detection and/or correction, redundancy, or the like, in certain embodiments.

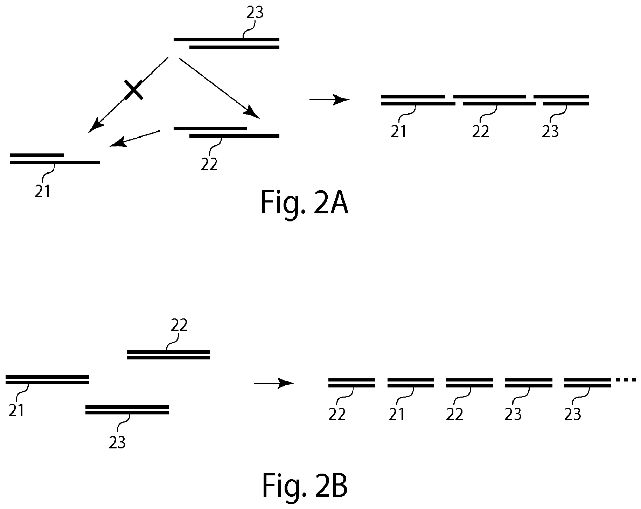

A nucleic acid tag may also include, in some cases, one or more connecting regions, which are ligated together. For example, the connecting regions may include "sticky ends," or overhangs of nucleic acids, such that only specific nucleic acids can be properly ligated together. For instance, as is shown in Fig. 2A, a first nucleic acid tag 21 (encoding a first condition) may include a first sticky end that is substantially complementary to a sticky end on second nucleic acid tag 22 but not to a sticky end on third nucleic acid tag 23; similarly, second nucleic acid 22 (encoding a second condition) may include a sticky end that is substantially complementary to a sticky end on third nucleic acid tag 23 (encoding a third condition) but not to the sticky end on first nucleic acid 21. Thus, upon exposure to suitable ligases, the first, second, and third nucleic acids may be joined or ligated together in an order suitable for subsequent study, without the nucleic acids being incorrectly ligated together in an incorrect order (e.g., a first nucleic acid being ligated to another first nucleic acid). Accordingly, by sequencing the final ligated nucleic acid, it can be determined that this nucleic acid was in a droplet or other entity exposed to the first, second, and third conditions.

However, it should be understood that in other embodiments, there may be no need to ensure that the nucleic acid tags are ligated together in a certain configuration or order. For example, as is shown in Fig. 2B, nucleic acids 21, 22, and 23 may be ligated together in any suitable order; and the resulting ligated nucleic acid may be analyzed to determine that that the nucleic acid encoded the conditions encoded by nucleic acids 21, 22, and 23.

The nucleic acid tag may also have any suitable length or number of nucleotides, depending on the application. For example, a nucleic acid tag may have a length shorter or longer than 10 nt, 30 nt, 50 nt, 100 nt, 300 nt, 500 nt, 1000 nt, 3000 nt, 5000 nt, or 10000 nt, etc. In some cases, other portions of the nucleic acid tag may also be used for other purposes, e.g., in addition to encoding conditions. For example, portions of the nucleic acid tag may be used to increase the bulk of the nucleic acid tag (e.g., using specific sequences or nonsense sequences), to facilitate handling (for example, a tag may include a poly-A tail), to increase selectivity of binding (e.g., as discussed below), to facilitate recognition by an enzyme (e.g., a suitable ligase), to facilitate identification, or the like.

As mentioned, in certain aspects of the invention, one or more conditions may be encoded using such tags, e.g., added to droplets or other entities exposed to such conditions. Thus, for example, a droplet may be exposed to a first condition and a first tag added to the droplet, then the droplet may be exposed to a second condition and a second tag added to the droplet, then the droplet may be exposed to a third condition and a third tag added to the droplet, then the droplet may be exposed to a fourth condition and a fourth tag added to the droplet, etc. Accordingly, any number of conditions may be present, e.g., the droplet or other entity may be exposed to 2 3, 4, 5, 6, 7, 8, 9, 10, or more conditions. The tags may also be combined together (e.g., ligated together) to produce a joined tag that encodes the history and/or identity of the droplet or entity, as previously discussed.

Any suitable conditions may be encoded by the tags. For example, in one set of embodiments, the identity of the droplet or other entity may be encoded using one or more tags. For example, each droplet or entity may be assigned a unique tag, or a unique combination of tags. As another example, the condition may be a species that a droplet or other entity is exposed to, internally and/or externally. Any of a wide variety of species may be encoded by a suitable tag. For example, the species may be a drug (or a suspected drug), a cell, a polymer, a peptide, a protein, an enzyme, a hormone, an antibiotic, a vitamin, a carbohydrate, a sugar, an antibody, a reagent, a gas, a dye, an ion, a virus, a bacterium, pH level (e.g., acidic or basic conditions), or the like. Thus, for example, a plurality of cells, or a plurality of cell types may be encoded using a unique tag, or a unique combination of tags. Any number of tags may be used to encode such conditions, depending on the application. For example, there may be at least 10, at least 30, at least 50, at least 100, at least 300, at least 500, at least 1,000, at least 3,000, at least 5,000, at least 10,000, at least 30,000, at least 50,000, at least 100,000, or more unique tags, e.g., substantially encoding a like number of suitable conditions such as any of those described herein. For instance, as a first condition, a library of droplets, containing a plurality of cells or cell types, may be encoded such that substantially each droplet contains one cell or one cell type, and an associated tag, such as a nucleic acid.

In addition, the condition may also be an external physical condition in some instances. For example, the droplet or other entity may be exposed to an external physical condition such as a certain or predetermined temperature, pressure, electrical condition (e.g., current, voltage, etc.), etc. and a suitable tag may be introduced into the droplet or entity before, during, or after exposure to the external physical condition. As yet another example, the condition may be a processing condition, for example, filtration, sedimentation, centrifugation, etc.

In one set of embodiments, the condition may be a condition used to lyse cells that may be contained within the droplets. For example, the condition could be exposure to a lysing chemical (e.g., pure water, a surfactant such as Triton-X or SDS, an enzyme such as lysozyme, lysostaphin, zymolase, cellulase, mutanolysin, glycanases, proteases, mannase, etc.), or a physical condition (e.g., ultrasound, ultraviolet light, mechanical agitation, etc.). In some cases, lysing a cell will cause the cell to release its contents, e.g., cellular nucleic acids, proteins, enzymes, sugars, etc. In some embodiments some of the cellular nucleic acids may also be ligated to one or more nucleic acids contained within the droplet. For example, in one set of embodiments, RNA transcripts typically produced within the cells may be released and then ligated to the nucleic acid tags.

Combinations of any of these and/or other conditions are also contemplated. For example, as noted above, a droplet may be exposed to a first condition encoded by a first tag, a second condition encoded by a second tag, a third condition encoded by a third tag, etc. The conditions may be the same or different. In addition, a droplet or other entity may be exposed to any number of conditions (e.g. 1, 2, 3, 4, 5, 6, etc.), and different droplets or entities need not be exposed to the same conditions, or the same number of conditions. In addition, in some embodiments, a plurality of droplets (or other entities) may be randomly exposed to a plurality of conditions, e.g., such that not all droplets are exposed to all conditions. As noted above, when a droplet or other entity is exposed to a condition, a nucleic acid encoding the condition may be added to the droplet or entity, such that the specific history of the droplet or entity need not be predetermined, and can be randomly determined. For instance, a plurality of initial, substantially identical droplets (or other entities) may be exposed to a plurality of second droplets containing different species (and/or one or more species at different concentrations), such that each initial droplet is randomly fused to one or more second droplets.

In addition, in some embodiments, droplets or other entities may be separated or sorted into various groups depending on various properties, and the tags within each group may be manipulated together and/or used to identify such droplet or entities having such properties. Thus, as non-limiting examples, a droplet (or other entity) may be sorted into a first group or a second group depending on whether a reaction occurred within the droplet or not, the droplet may be sorted based on a property of a cell or other species contained within the droplet, etc. The sorting may also occur into two or more groups.

In some cases, some or all of the groups of droplets (or other entities) may be analyzed, e.g., using the tags contained therein, to determine characteristics of the droplets or entities within those groups, and/or to determine characteristics of the droplets or entities within a group that separates that group from other groups. For instance, members of a group may have, in common, one or more tags, and/or one or more specific combinations of tags, that separates those group members from other group members, e.g., as was explained above with respect to Fig. 1C. Such tags may be used, for example, to identify or distinguish one or more conditions that result in a particular outcome from conditions that do not result in that outcome.

Thus, in one set of embodiments, a property of a droplet or other entity is determined, and the droplet or other entity is sorted based on that property. The property may also be a property of a species contained within the droplet or entity, such as a cell. The property may be any physical or chemical property that can be determined. For instance, properties such as fluorescence, transparency, density, size, volume, etc., of a droplet or other entity (or species contained therein) may be determined, and used for sorting purposes. In some cases, one or more reagents may also be added to the droplets or other entities, e.g., to start or facilitate a reaction that can be determined, e.g., for sorting purposes.

In some cases, the droplets or other entities may be burst or disrupted in order to access the tags. This may occur at any suitable time, e.g., before or after ligation or joining of the tags together. For example, droplets contained in a carrying fluid may be disrupted using techniques such as mechanical disruption or ultrasound. Similarly, entities on a surface may be disrupted using exposure to chemical agents or surfactants, or washed or rinsed to collect the tags.

The tags may then be determined to determine the identity and/or history of the droplet (or other entity), e.g., to determine conditions that the droplet or other entity was exposed to. Any suitable method can be used to determine the tags, depending on the type of tags used. For example, fluorescent particles may be determined using fluorescence measurements, or nucleic acids may be sequenced using a variety of techniques and instruments, many of which are readily available commercially. Examples of such techniques include, but are not limited to, chain-termination sequencing, sequencing-by-hybridization, Maxam-Gilbert sequencing, dye-terminator sequencing, chain-termination methods, Massively Parallel Signature Sequencing (Lynx Therapeutics), polony sequencing, pyrosequencing, sequencing by ligation, ion semiconductor sequencing, DNA nanoball sequencing, single-molecule real-time sequencing, nanopore sequencing, microfluidic Sanger sequencing, digital RNA sequencing ("digital RNA-seq"), etc.

In addition, in some cases, one or more other species may be associated with the tags, e.g., covalently bound or ligated thereto. For example, as previously discussed, in some cases, nucleic acids released by a lysed cell may be ligated to one or more tags. These may include, for example, chromosomal DNA, RNA transcripts, tRNA, mRNA, mitochondrial DNA, or the like. Such nucleic acids may be sequenced, in addition to sequencing the tags themselves, which may yield information about the nucleic acid profile of the cells, which can be associated with the tags, or the conditions that the corresponding droplet or cell was exposed to. Thus, as a non-limiting example, RNA transcripts from the cell may be ligated to one or more tags, which may be sequenced and correlated with conditions that the corresponding droplet or cell to determine information such as apoptosis gene signatures, growth arrest signatures, immune signatures, metabolic gene sets, or expression of genes that confer susceptibility or resistance to other known agents, etc.

Tags such as those described herein may be used in a variety of situations, for example, to screen or bioprospect for new drugs or other treatments, as a high-throughput screening technique, to study the effect various exposure conditions have on cells, or the like. In addition, it should be understood that the invention is not limited to only cell studies. For example, a chemical contained within a droplet may be exposed to a variety of different conditions (e.g., potential reactants or catalysts, reaction conditions, initiators, etc.) and the different conditions tagged for determining or optimizing the reactions that the chemical is able to participate in. In some cases, the chemical may be a biologically active chemical (e.g., a drug, a protein, a polymer, a carbohydrate, etc.), although in other cases, the chemical need not be biologically relevant. For example, the chemical may be a monomer or a polymer, a catalyst, a semiconductor material, etc.

As a non-limiting example, in one set of embodiments, a plurality of substantially identical cells can be exposed to a variety of substances (e.g., other cells, chemical compounds, soil samples, naturally-occurring samples, etc.), optionally under various physical conditions (e.g., various temperatures), to determine whether any of the substances have a beneficial or a detrimental effect on the cells. For example, the substantially identical cells may be cancer cells that are screened against a panel of substances to identify those substances that, alone or in combination, have anti-cancer or anti-tumor properties, or the substantially identical cells may be immune or other cells to which a certain activity is desired. The panel may include, for example, naturally-occurring compounds, synthetic compounds, compounds from a library, compounds sharing certain properties, etc. In some cases, the compounds may also be present at more than one concentration. Each member of the panel may be tagged as discussed herein, such that, upon exposure of a cell in a droplet (or other entity) to a panel member, an appropriate corresponding tag is added. The tags may also be ligated or otherwise joined together. Thus, for example, the substantially identical cells may be exposed to a variety of substances, in various combinations, with subsequent sorting of live cells from dead cells; the tags from the droplets containing the live cells (and/or the dead cells) may be separated and sequenced as discussed herein to determine those conditions or panel members which were able to kill the cells. In addition, the invention is not limited to only substantially identical cells. For example, in another set of embodiments, droplets containing different cells (or other species, e.g., chemicals) may be used.

Additional details regarding systems and methods for manipulating droplets in a microfluidic system follow, e.g., for determining droplets (or species within droplets), sorting droplets, etc. For example, various systems and methods for screening and/or sorting droplets are described in U.S. Patent Application Serial No. 11/360,845, filed February 23, 2006, entitled "Electronic Control of Fluidic Species," by Link, et al., published as U.S. Patent Application Publication No. 2007/000342 on January 4, 2007. As a non-limiting example, by applying (or removing) a first electric field (or a portion thereof), a droplet may be directed to a first region or channel; by applying (or removing) a second electric field to the device (or a portion thereof), the droplet may be directed to a second region or channel; by applying a third electric field to the device (or a portion thereof), the droplet may be directed to a third region or channel; etc., where the electric fields may differ in some way, for example, in intensity, direction, frequency, duration, etc.

In certain embodiments of the invention, sensors are provided that can sense and/or determine one or more characteristics of the fluidic droplets, and/or a characteristic of a portion of the fluidic system containing the fluidic droplet (e.g., the liquid surrounding the fluidic droplet) in such a manner as to allow the determination of one or more characteristics of the fluidic droplets. Characteristics determinable with respect to the droplet and usable in the invention can be identified by those of ordinary skill in the art. Non-limiting examples of such characteristics include fluorescence, spectroscopy (e.g., optical, infrared, ultraviolet, etc.), radioactivity, mass, volume, density, temperature, viscosity, pH, concentration of a substance, such as a biological substance (e.g., a protein, a nucleic acid, etc.), or the like.

In some cases, the sensor may be connected to a processor, which in turn, cause an operation to be performed on the fluidic droplet, for example, by sorting the droplet, adding or removing electric charge from the droplet, fusing the droplet with another droplet, splitting the droplet, causing mixing to occur within the droplet, etc., for example, as previously described. For instance, in response to a sensor measurement of a fluidic droplet, a processor may cause the fluidic droplet to be split, merged with a second fluidic droplet, etc.

One or more sensors and/or processors may be positioned to be in sensing communication with the fluidic droplet. "Sensing communication," as used herein, means that the sensor may be positioned anywhere such that the fluidic droplet within the fluidic system (e.g., within a channel), and/or a portion of the fluidic system containing the fluidic droplet may be sensed and/or determined in some fashion. For example, the sensor may be in sensing communication with the fluidic droplet and/or the portion of the fluidic system containing the fluidic droplet fluidly, optically or visually, thermally, pneumatically, electronically, or the like. The sensor can be positioned proximate the fluidic system, for example, embedded within or integrally connected to a wall of a channel, or positioned separately from the fluidic system but with physical, electrical, and/or optical communication with the fluidic system so as to be able to sense and/or determine the fluidic droplet and/or a portion of the fluidic system containing the fluidic droplet (e.g., a channel or a microchannel, a liquid containing the fluidic droplet, etc.). For example, a sensor may be free of any physical connection with a channel containing a droplet, but may be positioned so as to detect electromagnetic radiation arising from the droplet or the fluidic system, such as infrared, ultraviolet, or visible light. The electromagnetic radiation may be produced by the droplet, and/or may arise from other portions of the fluidic system (or externally of the fluidic system) and interact with the fluidic droplet and/or the portion of the fluidic system containing the fluidic droplet in such as a manner as to indicate one or more characteristics of the fluidic droplet, for example, through absorption, reflection, diffraction, refraction, fluorescence, phosphorescence, changes in polarity, phase changes, changes with respect to time, etc. As an example, a laser may be directed towards the fluidic droplet and/or the liquid surrounding the fluidic droplet, and the fluorescence of the fluidic droplet and/or the surrounding liquid may be determined. "Sensing communication," as used herein may also be direct or indirect. As an example, light from the fluidic droplet may be directed to a sensor, or directed first through a fiber optic system, a waveguide, etc., before being directed to a sensor.

Non-limiting examples of sensors useful in the invention include optical or electromagnetically-based systems. For example, the sensor may be a fluorescence sensor (e.g., stimulated by a laser), a microscopy system (which may include a camera or other recording device), or the like. As another example, the sensor may be an electronic sensor, e.g., a sensor able to determine an electric field or other electrical characteristic. For example, the sensor may detect capacitance, inductance, etc., of a fluidic droplet and/or the portion of the fluidic system containing the fluidic droplet.

As used herein, a "processor" or a "microprocessor" is any component or device able to receive a signal from one or more sensors, store the signal, and/or direct one or more responses (e.g., as described above), for example, by using a mathematical formula or an electronic or computational circuit. The signal may be any suitable signal indicative of the environmental factor determined by the sensor, for example a pneumatic signal, an electronic signal, an optical signal, a mechanical signal, etc.

In one set of embodiments, a fluidic droplet may be directed by creating an electric charge and/or an electric dipole on the droplet, and steering the droplet using an applied electric field, which may be an AC field, a DC field, etc. As an example, an electric field may be selectively applied and removed (or a different electric field may be applied, e.g., a reversed electric field) as needed to direct the fluidic droplet to a particular region. The electric field may be selectively applied and removed as needed, in some embodiments, without substantially altering the flow of the liquid containing the fluidic droplet. For example, a liquid may flow on a substantially steady-state basis (i.e., the average flowrate of the liquid containing the fluidic droplet deviates by less than 20% or less than 15% of the steady-state flow or the expected value of the flow of liquid with respect to time, and in some cases, the average flowrate may deviate less than 10% or less than 5%) or other predetermined basis through a fluidic system of the description (e.g., through a channel or a microchannel), and fluidic droplets contained within the liquid may be directed to various regions, e.g., using an electric field, without substantially altering the flow of the liquid through the fluidic system.

In some embodiments, the fluidic droplets may be screened or sorted within a fluidic system of the description by altering the flow of the liquid containing the droplets. For instance, in one set of embodiments, a fluidic droplet may be steered or sorted by directing the liquid surrounding the fluidic droplet into a first channel, a second channel, etc.

In another set of embodiments, pressure within a fluidic system, for example, within different channels or within different portions of a channel, can be controlled to direct the flow of fluidic droplets. For example, a droplet can be directed toward a channel junction including multiple options for further direction of flow (e.g., directed toward a branch, or fork, in a channel defining optional downstream flow channels). Pressure within one or more of the optional downstream flow channels can be controlled to direct the droplet selectively into one of the channels, and changes in pressure can be effected on the order of the time required for successive droplets to reach the junction, such that the downstream flow path of each successive droplet can be independently controlled. In one arrangement, the expansion and/or contraction of liquid reservoirs may be used to steer or sort a fluidic droplet into a channel, e.g., by causing directed movement of the liquid containing the fluidic droplet. The liquid reservoirs may be positioned such that, when activated, the movement of liquid caused by the activated reservoirs causes the liquid to flow in a preferred direction, carrying the fluidic droplet in that preferred direction. For instance, the expansion of a liquid reservoir may cause a flow of liquid towards the reservoir, while the contraction of a liquid reservoir may cause a flow of liquid away from the reservoir. In some cases, the expansion and/or contraction of the liquid reservoir may be combined with other flow-controlling devices and methods, e.g., as described herein. Non-limiting examples of devices able to cause the expansion and/or contraction of a liquid reservoir include pistons and piezoelectric components. In some cases, piezoelectric components may be particularly useful due to their relatively rapid response times, e.g., in response to an electrical signal. In some embodiments, the fluidic droplets may be sorted into more than two channels.

As mentioned, certain embodiments are generally directed to systems and methods for sorting fluidic droplets in a liquid, and in some cases, at relatively high rates. For example, a property of a droplet may be sensed and/or determined in some fashion (e.g., as further described herein), then the droplet may be directed towards a particular region of the device, such as a microfluidic channel, for example, for sorting purposes. In some cases, high sorting speeds may be achievable using certain systems and methods of the description. For instance, at least about 10 droplets per second may be determined and/or sorted in some cases, and in other cases, at least about 20 droplets per second, at least about 30 droplets per second, at least about 100 droplets per second, at least about 200 droplets per second, at least about 300 droplets per second, at least about 500 droplets per second, at least about 750 droplets per second, at least about 1,000 droplets per second, at least about 1,500 droplets per second, at least about 2,000 droplets per second, at least about 3,000 droplets per second, at least about 5,000 droplets per second, at least about 7,500 droplets per second, at least about 10,000 droplets per second, at least about 15,000 droplets per second, at least about 20,000 droplets per second, at least about 30,000 droplets per second, at least about 50,000 droplets per second, at least about 75,000 droplets per second, at least about 100,000 droplets per second, at least about 150,000 droplets per second, at least about 200,000 droplets per second, at least about 300,000 droplets per second, at least about 500,000 droplets per second, at least about 750,000 droplets per second, at least about 1,000,000 droplets per second, at least about 1,500,000 droplets per second, at least about 2,000,000 or more droplets per second, or at least about 3,000,000 or more droplets per second may be determined and/or sorted.

In some aspects, a population of relatively small droplets may be used. In certain embodiments, as non-limiting examples, the average diameter of the droplets may be less than about 1 mm, less than about 500 micrometers, less than about 300 micrometers, less than about 200 micrometers, less than about 100 micrometers, less than about 75 micrometers, less than about 50 micrometers, less than about 30 micrometers, less than about 25 micrometers, less than about 20 micrometers, less than about 15 micrometers, less than about 10 micrometers, less than about 5 micrometers, less than about 3 micrometers, less than about 2 micrometers, less than about 1 micrometer, less than about 500 nm, less than about 300 nm, less than about 100 nm, or less than about 50 nm. The average diameter of the droplets may also be at least about 30 nm, at least about 50 nm, at least about 100 nm, at least about 300 nm, at least about 500 nm, at least about 1 micrometer, at least about 2 micrometers, at least about 3 micrometers, at least about 5 micrometers, at least about 10 micrometers, at least about 15 micrometers, or at least about 20 micrometers in certain cases. The "average diameter" of a population of droplets is the arithmetic average of the diameters of the droplets.

In some embodiments, the droplets may be of substantially the same shape and/or size (i.e., "monodisperse"), or of different shapes and/or sizes, depending on the particular application. In some cases, the droplets may have a homogenous distribution of cross-sectional diameters, i.e., the droplets may have a distribution of diameters such that no more than about 5%, no more than about 2%, or no more than about 1% of the droplets have a diameter less than about 90% (or less than about 95%, or less than about 99%) and/or greater than about 110% (or greater than about 105%, or greater than about 101%) of the overall average diameter of the plurality of droplets. Some techniques for producing homogenous distributions of cross-sectional diameters of droplets are disclosed in International Patent Application No. PCT/US2004/010903, filed April 9, 2004, entitled "Formation and Control of Fluidic Species," by Link et al., published as WO 2004/091763 on October 28, 2004.

Those of ordinary skill in the art will be able to determine the average diameter of a population of droplets, for example, using laser light scattering or other known techniques. The droplets so formed can be spherical, or non-spherical in certain cases. The diameter of a droplet, in a non-spherical droplet, may be taken as the diameter of a perfect mathematical sphere having the same volume as the non-spherical droplet.

In some embodiments, one or more droplets may be created within a channel by creating an electric charge on a fluid surrounded by a liquid, which may cause the fluid to separate into individual droplets within the liquid. In some embodiments, an electric field may be applied to the fluid to cause droplet formation to occur. The fluid can be present as a series of individual charged and/or electrically inducible droplets within the liquid. Electric charge may be created in the fluid within the liquid using any suitable technique, for example, by placing the fluid within an electric field (which may be AC, DC, etc.), and/or causing a reaction to occur that causes the fluid to have an electric charge.

The electric field, in some embodiments, is generated from an electric field generator, i.e., a device or system able to create an electric field that can be applied to the fluid. The electric field generator may produce an AC field (i.e., one that varies periodically with respect to time, for example, sinusoidally, sawtooth, square, etc.), a DC field (i.e., one that is constant with respect to time), a pulsed field, etc. Techniques for producing a suitable electric field (which may be AC, DC, etc.) are known to those of ordinary skill in the art. For example, in one embodiment, an electric field is produced by applying voltage across a pair of electrodes, which may be positioned proximate a channel such that at least a portion of the electric field interacts with the channel. The electrodes can be fashioned from any suitable electrode material or materials known to those of ordinary skill in the art, including, but not limited to, silver, gold, copper, carbon, platinum, copper, tungsten, tin, cadmium, nickel, indium tin oxide ("ITO"), etc., as well as combinations thereof.

In another set of embodiments, droplets of fluid can be created from a fluid surrounded by a liquid within a channel by altering the channel dimensions in a manner that is able to induce the fluid to form individual droplets. The channel may, for example, be a channel that expands relative to the direction of flow, e.g., such that the fluid does not adhere to the channel walls and forms individual droplets instead, or a channel that narrows relative to the direction of flow, e.g., such that the fluid is forced to coalesce into individual droplets. In some cases, the channel dimensions may be altered with respect to time (for example, mechanically or electromechanically, pneumatically, etc.) in such a manner as to cause the formation of individual droplets to occur. For example, the channel may be mechanically contracted ("squeezed") to cause droplet formation, or a fluid stream may be mechanically disrupted to cause droplet formation, for example, through the use of moving baffles, rotating blades, or the like.

Certain embodiments are generally directed to systems and methods for splitting a droplet into two or more droplets. For example, a droplet can be split using an applied electric field. The droplet may have a greater electrical conductivity than the surrounding liquid, and, in some cases, the droplet may be neutrally charged. In certain embodiments, in an applied electric field, electric charge may be urged to migrate from the interior of the droplet to the surface to be distributed thereon, which may thereby cancel the electric field experienced in the interior of the droplet. In some embodiments, the electric charge on the surface of the droplet may also experience a force due to the applied electric field, which causes charges having opposite polarities to migrate in opposite directions. The charge migration may, in some cases, cause the drop to be pulled apart into two separate droplets.

Some embodiments of the description generally relate to systems and methods for fusing or coalescing two or more droplets into one droplet, e.g., where the two or more droplets ordinarily are unable to fuse or coalesce, for example, due to composition, surface tension, droplet size, the presence or absence of surfactants, etc. In certain cases, the surface tension of the droplets, relative to the size of the droplets, may also prevent fusion or coalescence of the droplets from occurring.

As a non-limiting example, two droplets can be given opposite electric charges (i.e., positive and negative charges, not necessarily of the same magnitude), which can increase the electrical interaction of the two droplets such that fusion or coalescence of the droplets can occur due to their opposite electric charges. For instance, an electric field may be applied to the droplets, the droplets may be passed through a capacitor, a chemical reaction may cause the droplets to become charged, etc. The droplets, in some cases, may not be able to fuse even if a surfactant is applied to lower the surface tension of the droplets. However, if the droplets are electrically charged with opposite charges (which can be, but are not necessarily of, the same magnitude), the droplets may be able to fuse or coalesce. As another example, the droplets may not necessarily be given opposite electric charges (and, in some cases, may not be given any electric charge), and are fused through the use of dipoles induced in the droplets that causes the droplets to coalesce. Also, the two or more droplets allowed to coalesce are not necessarily required to meet "head-on." Any angle of contact, so long as at least some fusion of the droplets initially occurs, is sufficient. See also, e.g., U.S. Patent Application Serial No. 11/698,298, filed January 24, 2007, entitled "Fluidic Droplet Coalescence," by Ahn, *et al.,* published as U.S. Patent Application Publication No. 2007/0195127 on August 23, 2007.

In one set of embodiments, a fluid may be injected into a droplet. The fluid may be microinjected into the droplet in some cases, e.g., using a microneedle or other such device. In other cases, the fluid may be injected directly into a droplet using a fluidic channel as the droplet comes into contact with the fluidic channel. Other techniques of fluid injection are disclosed in, e.g., International Patent Application No. PCT/US2010/040006, filed June 25, 2010, entitled "Fluid Injection," by Weitz, *et al.,* published as WO 2010/151776 on December 29, 2010; or International Patent Application No. PCT/US2009/006649, filed December 18, 2009, entitled "Particle-Assisted Nucleic Acid Sequencing," by Weitz, *et al.,* published as WO 2010/080134 on July 15, 2010.

A variety of materials and methods, according to certain aspects of the description, can be used to form articles or components such as those described herein, e.g., channels such as microfluidic channels, chambers, etc. For example, various articles or components can be formed from solid materials, in which the channels can be formed via micromachining, film deposition processes such as spin coating and chemical vapor deposition, laser fabrication, photolithographic techniques, etching methods including wet chemical or plasma processes, and the like. See, for example, Scientific American, 248:44-55, 1983 (Angell, *et al*).

In one set of embodiments, various structures or components of the articles described herein can be formed of a polymer, for example, an elastomeric polymer such as polydimethylsiloxane ("PDMS"), polytetrafluoroethylene ("PTFE" or Teflon®), or the like. For instance, according to one embodiment, a microfluidic channel may be implemented by fabricating the fluidic system separately using PDMS or other soft lithography techniques (details of soft lithography techniques suitable for this embodiment are discussed in the references entitled "Soft Lithography," by Younan Xia and George M. Whitesides, published in the Annual Review of Material Science, 1998, Vol. 28, pages 153-184, and "Soft Lithography in Biology and Biochemistry," by George M. Whitesides, Emanuele Ostuni, Shuichi Takayama, Xingyu Jiang and Donald E. Ingber, published in the Annual Review of Biomedical Engineering, 2001, Vol. 3, pages 335-373.

Other examples of potentially suitable polymers include, but are not limited to, polyethylene terephthalate (PET), polyacrylate, polymethacrylate, polycarbonate, polystyrene, polyethylene, polypropylene, polyvinylchloride, cyclic olefin copolymer (COC), polytetrafluoroethylene, a fluorinated polymer, a silicone such as polydimethylsiloxane, polyvinylidene chloride, bis-benzocyclobutene ("BCB"), a polyimide, a fluorinated derivative of a polyimide, or the like. Combinations, copolymers, or blends involving polymers including those described above are also envisioned. The device may also be formed from composite materials, for example, a composite of a polymer and a semiconductor material.

In some embodiments, various structures or components of the article are fabricated from polymeric and/or flexible and/or elastomeric materials, and can be conveniently formed of a hardenable fluid, facilitating fabrication via molding (e.g. replica molding, injection molding, cast molding, etc.). The hardenable fluid can be essentially any fluid that can be induced to solidify, or that spontaneously solidifies, into a solid capable of containing and/or transporting fluids contemplated for use in and with the fluidic network. In one embodiment, the hardenable fluid comprises a polymeric liquid or a liquid polymeric precursor (i.e. a "prepolymer"). Suitable polymeric liquids can include, for example, thermoplastic polymers, thermoset polymers, waxes, metals, or mixtures or composites thereof heated above their melting point. As another example, a suitable polymeric liquid may include a solution of one or more polymers in a suitable solvent, which solution forms a solid polymeric material upon removal of the solvent, for example, by evaporation. Such polymeric materials, which can be solidified from, for example, a melt state or by solvent evaporation, are well known to those of ordinary skill in the art. A variety of polymeric materials, many of which are elastomeric, are suitable, and are also suitable for forming molds or mold masters, for embodiments where one or both of the mold masters is composed of an elastomeric material. A non-limiting list of examples of such polymers includes polymers of the general classes of silicone polymers, epoxy polymers, and acrylate polymers. Epoxy polymers are characterized by the presence of a three-membered cyclic ether group commonly referred to as an epoxy group, 1,2-epoxide, or oxirane. For example, diglycidyl ethers of bisphenol A can be used, in addition to compounds based on aromatic amine, triazine, and cycloaliphatic backbones. Another example includes the well-known Novolac polymers. Non-limiting examples of silicone elastomers suitable for use according to the invention include those formed from precursors including the chlorosilanes such as methylchlorosilanes, ethylchlorosilanes, phenylchlorosilanes, dodecyltrichlorosilanes, etc.

Silicone polymers are used in certain embodiments, for example, the silicone elastomer polydimethylsiloxane. Non-limiting examples of PDMS polymers include those sold under the trademark Sylgard by Dow Chemical Co., Midland, MI, and particularly Sylgard 182, Sylgard 184, and Sylgard 186. Silicone polymers including PDMS have several beneficial properties simplifying fabrication of various structures of the description. For instance, such materials are inexpensive, readily available, and can be solidified from a prepolymeric liquid via curing with heat. For example, PDMSs are typically curable by exposure of the prepolymeric liquid to temperatures of about, for example, about 65 °C to about 75 °C for exposure times of, for example, about an hour. Also, silicone polymers, such as PDMS, can be elastomeric and thus may be useful for forming very small features with relatively high aspect ratios, necessary in certain embodiments of the description. Flexible (e.g., elastomeric) molds or masters can be advantageous in this regard.

One advantage of forming structures such as microfluidic structures or channels from silicone polymers, such as PDMS, is the ability of such polymers to be oxidized, for example by exposure to an oxygen-containing plasma such as an air plasma, so that the oxidized structures contain, at their surface, chemical groups capable of cross-linking to other oxidized silicone polymer surfaces or to the oxidized surfaces of a variety of other polymeric and non-polymeric materials. Thus, structures can be fabricated and then oxidized and essentially irreversibly sealed to other silicone polymer surfaces, or to the surfaces of other substrates reactive with the oxidized silicone polymer surfaces, without the need for separate adhesives or other sealing means. In most cases, sealing can be completed simply by contacting an oxidized silicone surface to another surface without the need to apply auxiliary pressure to form the seal. That is, the pre-oxidized silicone surface acts as a contact adhesive against suitable mating surfaces. Specifically, in addition to being irreversibly sealable to itself, oxidized silicone such as oxidized PDMS can also be sealed irreversibly to a range of oxidized materials other than itself including, for example, glass, silicon, silicon oxide, quartz, silicon nitride, polyethylene, polystyrene, glassy carbon, and epoxy polymers, which have been oxidized in a similar fashion to the PDMS surface (for example, via exposure to an oxygen-containing plasma). Oxidation and sealing methods useful in the context of the present description, as well as overall molding techniques, are described in the art, for example, in an article entitled "Rapid Prototyping of Microfluidic Systems and Polydimethylsiloxane," Anal. Chem., 70:474-480, 1998 (Duffy *et al.*).

Thus, in certain embodiments, the design and/or fabrication of the article may be relatively simple, e.g., by using relatively well-known soft lithography and other techniques such as those described herein. In addition, in some embodiments, rapid and/or customized design of the article is possible, for example, in terms of geometry. In one set of embodiments, the article may be produced to be disposable, for example, in embodiments where the article is used with substances that are radioactive, toxic, poisonous, reactive, biohazardous, etc., and/or where the profile of the substance (e.g., the toxicology profile, the radioactivity profile, etc.) is unknown. Another advantage to forming channels or other structures (or interior, fluid-contacting surfaces) from oxidized silicone polymers is that these surfaces can be much more hydrophilic than the surfaces of typical elastomeric polymers (where a hydrophilic interior surface is desired). Such hydrophilic channel surfaces can thus be more easily filled and wetted with aqueous solutions than can structures comprised of typical, unoxidized elastomeric polymers or other hydrophobic materials.referred

The following documents are referred to in their entirety for all purposes: Int. Pat. Apl. Pub. No. WO 2004/091763, entitled "Formation and Control of Fluidic Species," by Link *et al.;* Int. Pat. Apl. Pub. No. WO 2004/002627, entitled "Method and Apparatus for Fluid Dispersion," by Stone *et al.;* Int. Pat. Apl. Pub. No. WO 2006/096571, entitled "Method and Apparatus for Forming Multiple Emulsions," by Weitz *et al.;* Int. Pat. Apl. Pub. No. WO 2005/021151, entitled "Electronic Control of Fluidic Species," by Link *et al.;* Int. Pat. Apl. Pub. No. WO 2011/056546, entitled "Droplet Creation Techniques," by Weitz, *et al.;* Int. Pat. Apl. Pub. No. WO 2010/033200, entitled "Creation of Libraries of Droplets and Related Species," by Weitz, *et al.;* U.S. Pat. Apl. Pub. No. 2012-0132288, entitled "Fluid Injection," by Weitz, *et al.;* Int. Pat. Apl. Pub. No. WO 2008/109176, entitled "Assay And Other Reactions Involving Droplets," by Agresti, *et al.;* and Int. Pat. Apl. Pub. No. WO 2010/151776, entitled "Fluid Injection," by Weitz et al.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims, the invention may be practiced otherwise than as specifically described and claimed. The present disclosure is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

## Claims

1. A method for determining the history of a droplet, comprising exposing a droplet to a first condition and adding a first nucleic acid to the droplet by fusing the droplet to a second droplet containing the first nucleic acid, wherein the first nucleic acid encodes the first condition; exposing the droplet to a second condition and adding a second nucleic acid to the droplet by fusing the droplet to a third droplet containing the second nucleic acid, wherein the second nucleic acid encodes the second condition; and ligating the first nucleic acid and the second nucleic acid together.

2. The method of claim 1, wherein the second droplet:
(a) is fused to the droplet via dipoles induced in the droplets; and/or
(b) is fused to the droplet via opposite electric charges placed on the droplets.

3. The method of any one of claims 1-2, wherein exposing the droplet to the first condition comprises:
(a) exposing the droplet to an external physical condition, preferably wherein exposing the droplet to an external physical condition comprises exposing the droplet to a predetermined temperature and/or a predetermined pressure; and/or
(b) fusing the droplet to a second droplet having a pH of less than 7 and/or a pH of greater than 7.

4. The method of any one of claims 1-3, wherein the droplet:
(a) has an average cross-sectional diameter of less than about 1 mm; and/or
(b) is one of a plurality of droplets having a distribution of diameters such that no more than about 5% of the droplets have a diameter less than about 90% or greater than about 110% of the overall average diameter of the plurality of droplets; and/or
(c) is contained within a microfluidic channel.

5. The method of any one of claims 1-4, further comprising exposing the droplet to a third condition and adding a third nucleic acid to the droplet, wherein the third nucleic acid encodes the third condition, preferably further comprising attaching the third nucleic acid to one or both of the first nucleic acid and the second nucleic acid.

6. The method of any one of claims 1-5, further comprising determining a property of the droplet, preferably comprising sorting the droplet based on the property, and/or wherein the property is:
(a) fluorescence; and/or
(b) the average cross-sectional diameter of the droplet; and/or
(c) light absorption; and/or
(d) the concentration of an agent contained within the droplet; and/or
(e) the condition of a cell contained within the droplet, preferably wherein the property is whether the cell is alive or dead and/or the property is a concentration of an agent produced and/or consumed by the cell.

7. The method of any one of claims 1-6, further comprising separating the attached first nucleic acid and second nucleic acid from the droplet.

8. The method of any one of claims 1-7, further comprising bursting the droplet, preferably wherein the droplet is burst:
(a) after attaching the first nucleic acid and the second nucleic acid; and/or
(b) by exposing the droplet to ultrasound.

9. The method of any one of claims 1-8, wherein the droplet contains a cell, preferably:
(a) wherein the cell is a human cell; and/or
(b) wherein the cell is a cancer cell; and/or
(c) wherein the cell is an immune cell; and/or
(d) wherein the cell is a bacterial cell; and/or
(e) wherein the cell is naturally-occurring cell; and
wherein the first condition is:
(a) the cell's type; and/or
(b) exposure to a drug suspected of being capable of interacting with the cell; and/or
(c) exposure to a putative cytotoxic drug.

10. The method of any one of claims 1-9, further comprising sequencing the attached first and second nucleic acids.

## Patentansprüche

1. Verfahren zum Bestimmen der Vorgeschichte eines Tröpfchens, umfassend das Aussetzen eines Tröpfchens einer ersten Bedingung und das Hinzufügen einer ersten Nukleinsäure zu dem Tröpfchen durch das Fusionieren des Tröpfchens mit einem die erste Nukleinsäure beinhaltendem zweiten Tröpfchen, wobei die erste Nukleinsäure die erste Bedingung codiert; das Aussetzen des Tröpfchens einer zweiten Bedingung und das Hinzufügen einer zweiten Nukleinsäure zu dem Tröpfchen durch das Fusionieren des Tröpfchens mit einem die zweiten Nukleinsäure beinhaltendem dritten Tröpfchen, wobei die zweite Nukleinsäure die zweite Bedingung codiert; und das Ligieren der ersten Nukleinsäure mit der zweiten Nukleinsäure.

2. Verfahren gemäß Anspruch 1, wobei das zweite Tröpfchen:
(a) mit dem Tröpfchen mittels in den Tröpfchen induzierter Dipole fusioniert wird; und/oder
(b) mit dem Tröpfchen durch auf den Tröpfchen platzierten gegensätzlichen elektrischen Ladungen fusioniert wird.

3. Verfahren gemäß irgendeinem der Ansprüche 1-2, wobei das Aussetzen des Tröpfchens der ersten Bedingung umfasst:
(a) das Aussetzen des Tröpfchens einer externen physikalischen Bedingung, wobei bevorzugt das Aussetzen des Tröpfchens einer externen physikalischen Bedingung das Aussetzen des Tröpfchens einer vorbestimmten Temperatur und/oder eines vorbestimmten Drucks umfasst; und/oder
(b) das Fusionieren des Tröpfchens mit einem zweiten Tröpfchen, welches einen pH-Wert von weniger als 7 und/oder einen pH-Wert von größer als 7 aufweist.

4. Verfahren gemäß irgendeinem der Ansprüche 1-3, wobei das Tröpfchen:
(a) einen durchschnittlichen Querschnittsdurchmesser von weniger als ungefähr 1 mm hat; und/oder
(b) eines einer Vielzahl von Tröpfchen ist, welche eine Verteilung von Durchmessern hat, so dass nicht mehr als ungefähr 5% der Tröpfchen einen Durchmesser von weniger als ungefähr 90% oder mehr als ungefähr 110% des insgesamt durchschnittlichen Durchmessers der Vielzahl von Tröpfchen haben; und/oder
(c) innerhalb eines mikrofluidischen Kanals enthalten ist.

5. Verfahren gemäß irgendeinem der Ansprüche 1-4, weitergehend umfassend das Aussetzen des Tröpfchens einer dritten Bedingung und das Hinzufügen einer dritten Nukleinsäure zum Tröpfchen, wobei die dritte Nukleinsäure die dritte Bedingung codiert, bevorzugt weitergehend umfassend das Anheften der dritten Nukleinsäure an eine oder beide der ersten Nukleinsäure und der zweiten Nukleinsäure.

6. Verfahren gemäß irgendeinem der Ansprüche 1-5, weitergehend umfassend das Bestimmen einer Eigenschaft des Tröpfchens, bevorzugt umfassend das Sortieren des Tröpfchens basierend auf der Eigenschaft, und/oder wobei die Eigenschaft ist:
(a) Fluoreszenz; und/oder
(b) der durchschnittliche Querschnittsdurchmesser des Tröpfchens; und/oder
(c) Lichtabsorption; und/oder
(d) die Konzentration eines in dem Tröpfchen enthaltenen Agens; und/oder
(e) der Zustand einer in dem Tröpfchen enthaltenen Zelle, wobei bevorzugt die Eigenschaft ist, ob die Zelle lebend oder tot ist, und/oder die Eigenschaft eine Konzentration eines von der Zelle produzierten und/oder konsumierten Agens ist.

7. Verfahren gemäß irgendeinem der Ansprüche 1-6, weitergehend umfassend das Separieren der angehefteten ersten Nukleinsäure und zweiten Nukleinsäure von dem Tröpfchen.

8. Verfahren gemäß irgendeinem der Ansprüche 1-7, weitergehend umfassend das Platzen des Tröpfchens, bevorzugt wobei das Tröpfchen geplatzt wird:
(a) nach dem Anheften der ersten Nukleinsäure und der zweiten Nukleinsäure; und/oder
(b) durch das Aussetzen des Tröpfchens an Ultraschall.

9. Verfahren gemäß irgendeinem der Ansprüche 1-8, wobei das Tröpfchen eine Zelle enthält, bevorzugt:
(a) wobei die Zelle eine menschliche Zelle ist; und/oder
(b) wobei die Zelle eine Krebszelle ist; und/oder
(c) wobei die Zelle eine Immunzelle ist; und/oder
(d) wobei die Zelle eine bakterielle Zelle ist; und/oder
(e) wobei die Zelle (eine) natürlich auftretende Zelle ist;
und
wobei die erste Bedingung ist:
(a) der Typ der Zelle; und/oder
(b) Exposition gegenüber einem Arzneimittel, welches vermutet wird, die Fähigkeit zu haben, mit der Zelle zu interagieren; und/oder
(c) Exposition gegenüber einem putativ zytotoxischen Arzneimittel.

10. Verfahren gemäß irgendeinem der Ansprüche 1-9, weitergehend umfassend das Sequenzieren der angehefteten ersten und zweiten Nukleinsäuren.

## Revendications

1. Procédé de détermination de l'historique d'une gouttelette, comprenant l'exposition d'une gouttelette à une première condition et l'ajout d'un premier acide nucléique à la gouttelette en fusionnant la gouttelette à une deuxième gouttelette contenant le premier acide nucléique, dans lequel le premier acide nucléique code pour la première condition ; l'exposition de la gouttelette à une deuxième condition et l'ajout d'un deuxième acide nucléique à la gouttelette en fusionnant la gouttelette à une troisième gouttelette contenant le deuxième acide nucléique, dans lequel le deuxième acide nucléique code pour la deuxième condition ; et la ligature du premier acide nucléique et du deuxième acide nucléique ensemble.

2. Procédé selon la revendication 1, dans lequel la deuxième gouttelette :
(a) est fusionnée à la gouttelette par l'intermédiaire de dipôles induits dans les gouttelettes ; et/ou
(b) est fusionnée à la gouttelette par l'intermédiaire de charges électriques opposées placées sur les gouttelettes.

3. Procédé selon l'une quelconque des revendications 1 ou 2, dans lequel l'exposition de la gouttelette à la première condition comprend :
(a) l'exposition de la gouttelette à une condition physique externe, de préférence dans lequel l'exposition de la gouttelette à une condition physique externe comprend l'exposition de la gouttelette à une température prédéterminée et/ou à une pression prédéterminée ; et/ou
(b) la fusion de la gouttelette à une deuxième gouttelette présentant un pH inférieur à 7 et/ou un pH supérieur à 7.

4. Procédé selon l'une quelconque des revendications 1 à 3, dans lequel la gouttelette :
(a) présente un diamètre moyen en coupe inférieur à environ 1 mm ; et/ou
(b) est l'une d'une pluralité de gouttelettes présentant une distribution de diamètres de sorte que pas plus d'environ 5 % des gouttelettes présentent un diamètre inférieur à environ 90 % ou supérieur à environ 110 % du diamètre moyen global de la pluralité de gouttelettes ; et/ou
(c) est contenue au sein d'un canal microfluidique.

5. Procédé selon l'une quelconque des revendications 1 à 4, comprenant en outre l'exposition de la gouttelette à une troisième condition et l'ajout d'un troisième acide nucléique à la gouttelette, dans lequel le troisième acide nucléique code pour la troisième condition, de préférence comprenant en outre la fixation du troisième acide nucléique à l'un et/ou l'autre du premier acide nucléique et du deuxième acide nucléique.

6. Procédé selon l'une quelconque des revendications 1 à 5, comprenant en outre la détermination d'une propriété de la gouttelette, comprenant de préférence le tri de la gouttelette sur la base de la propriété, et/ou dans lequel la propriété est :
(a) une fluorescence ; et/ou
(b) le diamètre moyen en coupe de la gouttelette ; et/ou
(c) une absorption de lumière ; et/ou
(d) la concentration d'un agent contenu au sein de la gouttelette ; et/ou
(e) la condition d'une cellule contenue au sein de la gouttelette, de préférence dans lequel la propriété est si la cellule est vivante ou morte et/ou la propriété est une concentration d'un agent produit et/ou consommé par la cellule.

7. Procédé selon l'une quelconque des revendications 1 à 6, comprenant en outre la séparation du premier acide nucléique et du deuxième acide nucléique fixés de la gouttelette.

8. Procédé selon l'une quelconque des revendications 1 à 7, comprenant en outre l'éclatement de la gouttelette, de préférence dans lequel la gouttelette est éclatée :
(a) après fixation du premier acide nucléique et du deuxième acide nucléique ; et/ou
(b) par exposition de la gouttelette à des ultrasons.

9. Procédé selon l'une quelconque des revendications 1 à 8, dans lequel la gouttelette contient une cellule, de préférence :
(a) dans lequel la cellule est une cellule humaine ; et/ou
(b) dans lequel la cellule est une cellule cancéreuse ; et/ou
(c) dans lequel la cellule est une cellule immunitaire ; et/ou
(d) dans lequel la cellule est une cellule bactérienne ; et/ou
(e) dans lequel la cellule est une cellule présente naturellement ; et
dans lequel la première condition est :
(a) le type de la cellule ; et/ou
(b) une exposition à un médicament soupçonné d'être capable d'interagir avec la cellule ; et/ou
(c) l'exposition à un médicament cytotoxique putatif.

10. Procédé selon l'une quelconque des revendications 1 à 9, comprenant en outre le séquençage des premier et deuxième acides nucléiques fixés.
